# EUROPEAN PATENT APPLICATION

(11) **EP 4 407 307 A1**
(43) Date of publication of application: **31.07.2024**
(21) Application number: 21959578.2
(22) Date of filing: 03.12.2021
(51) Int. Cl.: G01N 27/327, G01N 27/02, G01N 33/72, B01L 3/00

(54) **RED BLOOD CELL GLYCATION MEASUREMENT USING MECHANICAL AND ELECTRICAL CHARACTERISTICS, AND METHOD FOR MEASURING VALUES OF GLYCATED HEMOGLOBIN BY USING SAME AND APPARATUS FOR PERFORMING SAME**

(30) Priority: 29.09.2021 KR 20210128520
(71) Applicant: Orange Biomed Ltd.,Co, Daejeon 34136 (KR)
(72) Inventor: KO, Ung Hyeon, Seoul 04715 (KR); KANG, Seung Jin, Seoul 04715 (KR); PARK, Eun Young, Seoul 04715 (KR)
(74) Representative: BCKIP Part mbB
(86) International application number: PCT/KR2021/018280
(87) International publication number: WO 2023/054810

(57) **Abstract**

The present disclosure relates to a method of measuring the concentration of glycated A1c hemoglobin. A glycated hemoglobin measuring apparatus includes an inlet into which a collected blood is introduced, a microchannel having a predetermined width so that red blood cells in the blood introduced into the inlet pass in an individual unit, an outlet through which the red blood cells that have passed through the microchannel are discharged, and a plurality of set of electrodes formed in contact with the microchannel between the inlet and the outlet, wherein the set of electrodes include a plurality of electrodes arranged at a predetermined interval according to a passage direction of the red blood cells passing through the microchannel. According to the present disclosure, the degree of hardness of glycated red blood cells may be calculated through the time taken to pass through the minute channel and thus more easily determined, without chemical measuring equipment.

## Description

### [Technical Field]

The present disclosure relates to a method of measuring the concentration of glycated A1c hemoglobin.

### [Background Art]

A blood sugar test, which is generally performed for the diagnosis of diabetes, measures the level of sugar in the blood as a blood sugar level. However, the blood sugar level is a temporary value and may change before or after meals or due to other factors.

In contrast, a glycated hemoglobin test is measured numerically to determine a level of sugar linked to hemoglobin in red blood cells. The red blood cells are linked to sugar in the blood while the red blood cells exist in the blood, and the blood sugar level accumulated for three months, which is the average lifespan of the red blood cells, may be identified by measuring the glycated hemoglobin level. Therefore, the glycosylated hemoglobin test is relatively less affected by exercise status or food intake than other blood sugar tests.

That is, the glycated hemoglobin level is a more stable level than the blood sugar level and may be a reference for diagnosing diabetes, and a high glycated hemoglobin level makes the red blood cells relatively hard, which is a direct cause of diabetic complications due to an increase in blood viscosity.

However, most existing glycated hemoglobin measurement devices have no choice but to measure by using specific technology and equipment at the hospital and laboratory levels. Although continuous management of glycated hemoglobin is required in order to manage diabetes level and its prognosis, methods by which individual patients can manage themselves are very limited and have been recently developed. Like a blood sugar measurement device that has been popularized for home use, various methods have recently been devised (Korean Patent Registration Publication KR2281500 (registration date: July 20, 2021)) to measure glycated hemoglobin directly at home without visiting a clinic. However, since all of these techniques are based on biochemical methods, there are disadvantages in that the lifespan of a device is limited, a storage method is difficult, and a measurement accuracy is low depending on a storage condition or the skill of a user.

### [Disclosure]

### [Technical Problem]

An object of the present disclosure is to propose a method of determining a glycated hemoglobin level.

More specifically, the present disclosure relates to the development of a device for measuring glycated hemoglobin for home use using a microchannel manufacturing technology. More specifically, an object of the present disclosure is to propose a method of determining the degree of glycation by measuring a change in the physical characteristic of a glycated red blood cell.

### [Technical Solution]

According to an exemplary embodiment of the present disclosure, a glycated hemoglobin measuring apparatus includes an inlet into which a collected blood is introduced; a microchannel having a predetermined width so that red blood cells in the blood introduced into the inlet pass in an individual unit; an outlet through which the red blood cells that have passed through the microchannel are discharged; and a plurality of set of electrodes formed in contact with the microchannel between the inlet and the outlet, wherein the set of electrodes include a plurality of electrodes arranged at a predetermined interval according to a passage direction of the red blood cells passing through the microchannel.

The set of electrodes may include a first set of electrodes on an inlet side and a second set of electrodes on an outlet side, and an impedance between at least one pair of electrodes in the first set of electrodes and an impedance between at least one pair of electrodes in the second set of electrodes may change according to passage of the red blood cells.

Each of the first and second set of electrodes may include three continuous electrodes, and an electrical equilibrium state of a first impedance and a second impedance formed between two consecutive electrodes of the first set of electrodes, and an electrical equilibrium state of a third impedance and a fourth impedance formed between two consecutive electrodes of the second set of electrodes may change according to the passage of the red blood cells.

According to another exemplary embodiment of the present disclosure, a glycated hemoglobin measuring apparatus includes a time measuring unit measuring a microchannel passage time of red blood cells using a difference between a time based on the change of an impedance value as the red blood cells in blood pass through at a first node on a microchannel formed in contact with set of electrodes between a blood inlet and an outlet, and a time based on the change of an impedance value at a second node of the microchannel; and a glycated hemoglobin level calculating unit determining a degree of glycation of the red blood cells in the blood based on the measured time.

the set of electrodes may include a first set of electrodes on an inlet side and a second set of electrodes on an outlet side, and the time measuring unit may calculate the microchannel passage time of the red blood cells by a difference between a time based on the change of an impedance value of the first set of electrodes and a time based on the change of an impedance value of the second set of electrodes.

Each of the first and second set of electrodes may include a plurality of electrodes, and the time measuring unit may calculate the microchannel passage time of the red blood cells using a difference between a time based on the change of the impedance value when the red blood cells are positioned between at least one pair of electrodes in the first set of electrodes, and a time based on the change of the impedance value when the red blood cells are positioned between at least one pair of electrodes in the second set of electrodes.

The glycated hemoglobin level calculating unit may calculate the degree of glycation of the blood by using a proportional relationship between the microchannel passage time and glycation of the red blood cells in the blood.

The glycated hemoglobin measuring apparatus of may further include a glycated hemoglobin level correcting unit correcting the degree of glycation of the blood by using a reference glycated hemoglobin level of a user.

According to another exemplary embodiment of the present disclosure, a glycated hemoglobin measuring method includes measuring a time based on the change of an impedance value as red blood cells in blood pass through at a first node on a channel formed in contact with set of electrodes between a blood inlet and an outlet; measuring a time based on the change of an impedance value at a second node of the microchannel; measuring a microchannel passage time using a difference between a first impedance time based on the change of the first node and a second impedance time based on the change of the second node; and calculating a glycated hemoglobin level according to the measured microchannel passage time.

The first node may be determined as at least one position on a zone where a first set of electrodes on an inlet side and the microchannel contact each other, and the second node may be determined as at least one position on a zone where a second set of electrodes on an outlet side and the microchannel contact each other.

The first or second impedance time based on the change may be measured as a change in the impedance value as the red blood cells passing through the microchannel are positioned between a pair of electrodes in the first or second set of electrodes.

The calculating of the glycated hemoglobin level may include calculating a degree of glycation of the blood using a proportional relationship between the microchannel passage time and glycation of red blood cells in the blood.

The glycated hemoglobin measuring method may further include correcting the calculated degree of glycation of the blood using a reference glycated hemoglobin level of a user.

Each of the first and second set of electrodes may include three continuous electrodes, the measuring of the microchannel passage time may include measuring a change in the impedance value using a change in electrical equilibrium states of a first impedance and a second impedance formed between two continuous electrodes of the first set of electrodes, and electrical equilibrium states of a third impedance and a fourth impedance formed between two continuous electrodes of the second set of electrodes, and calculating the microchannel passage time by using a time based on the change of the impedance value.

### [Advantageous Effects]

According to the various exemplary embodiments of the present disclosure, the degree of glycation may be easily measured using the change in the physical characteristics of red blood cells according to the glycation of the red blood cells.

In addition, the degree of glycation may be determined more stably in response to external and human factors compared to the measurement equipment of biochemical technique by calculating the hardness of each individual red blood cell through the time taken to pass through a minute channel.

In addition, the present disclosure may recognize a minute electrical change occurred by the passage of the red blood cells through a simple circuit configuration, and through this, determine the degree of glycation of the red blood cells.

In addition, the present disclosure may be directly utilized for clinical diagnosis by correcting the glycated hemoglobin level measured by using an individual reference value.

In addition, the present disclosure may be popularized as a measuring device for home use through miniaturization of the measuring device.

### [Description of Drawings]

FIG. 1 is a diagram showing a structure of an apparatus for determining a glycated hemoglobin level according to the present disclosure.
FIG. 2 is a diagram illustrating a microchannel structure and a principle of the apparatus for determining the glycated hemoglobin level according to an embodiment of the present disclosure.
FIGS. 3 to 5 are diagrams illustrating an electrical structure for determining the physical level of red blood cells according to the present invention.
FIG. 6 is a diagram showing a structure of an apparatus for determining the glycated hemoglobin level according to the present disclosure.
FIG. 7 is a diagram showing a configuration of an apparatus for determining the glycated hemoglobin level according to the present disclosure.
FIG. 8 is a flowchart showing a method of determining the glycated hemoglobin level according to the present disclosure.
FIG. 9 is a diagram illustrating an example of patient data for managing the glycated hemoglobin level according to the present disclosure.

### [Best Mode]

The following description illustrates only a principle of the present disclosure. Therefore, those skilled in the art may invent various devices that implement the principle of the present disclosure and are included in the spirit and scope of the present disclosure although they are not clearly described or shown in the present specification. In addition, it is to be understood that all conditional terms and exemplary embodiments listed in the present specification are obviously intended only to allow those skilled in the art to understand a concept of the present disclosure in principle, and the present disclosure is not limited to the exemplary embodiments and states particularly listed as described above.

The above-mentioned objects, features, and advantages will become more obvious from the following detailed description provided in relation to the accompanying drawings. Therefore, those skilled in the art to which the present disclosure pertains may easily practice a technical idea of the present disclosure.

Further, in describing the present disclosure, in the case in which it is determined that a detailed description of a known technology associated with the present disclosure may unnecessarily make the gist of the present disclosure unclear, it will be omitted. Hereinafter, exemplary embodiments of the present disclosure will be described in detail with reference to the accompanying drawings.

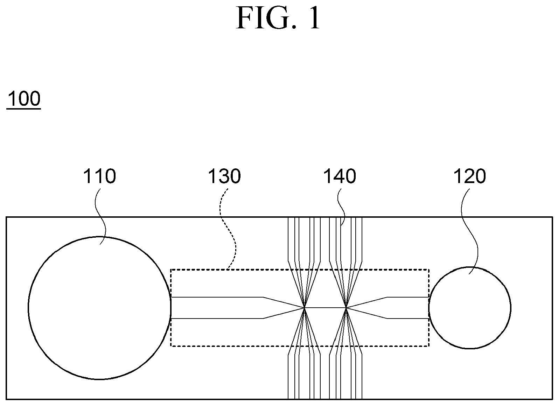

FIG. 1 is a diagram showing a structure of an apparatus 100 for determining a glycated hemoglobin level according to the present disclosure.

Referring to FIG. 1, the glycated hemoglobin measuring apparatus 100 according to the present disclosure may include an inlet 110 into which blood is introduced, a microchannel 130 through which red blood cells in the blood introduced into the inlet 110 pass, and an outlet 120 through which the red blood cells that have passed through the microchannel 130 is discharged.

In the present embodiment, the glycated hemoglobin measuring apparatus 100 as a plate-shaped chip may configure the microchannel 130 on a substrate between the inlet 110 and the outlet 120 opened to the outside, and set of electrodes 140 in which a plurality of patterned electrodes are in contact with each other and arranged may be formed in the microchannel 130 between the inlet 110 and the outlet 120. The inlet 110 may be configured to have a larger diameter than that of the outlet 120 so as to facilitate blood dripping, and the outlet 120 may induce the blood introduced into the inlet 110 to be discharged along the microchannel through a pump in a main body when combined with the main body.

The set of electrodes 140 may be respectively formed in the microchannel on the side of the inlet 110 and the side of the outlet 120, and a plurality of electrodes may be disposed on each set of electrodes 140 at a predetermined interval.

Specifically, the electrodes may be disposed in a shape perpendicular to a flow direction of the blood in the microchannel, and may be arranged at an equal interval according to the flow direction. Furthermore, distances between the electrodes may be set with respect to the size of one red blood cell in order to distinguish red blood cells passing through the microchannel.

In the present embodiment, the electrodes in the set of electrodes 140 may be configured in two groups of three each, and an electrolyte allows blood to pass through between the three electrodes in the same group, so that current may flow.

The glycated hemoglobin measuring apparatus 100 according to the present embodiment determines the degree of glycation by using a microchannel passage time of the red blood cells in the blood, and uses an impedance change between the electrodes to determine passage time nodes of the minute red blood cells. This will be described below in more detail.

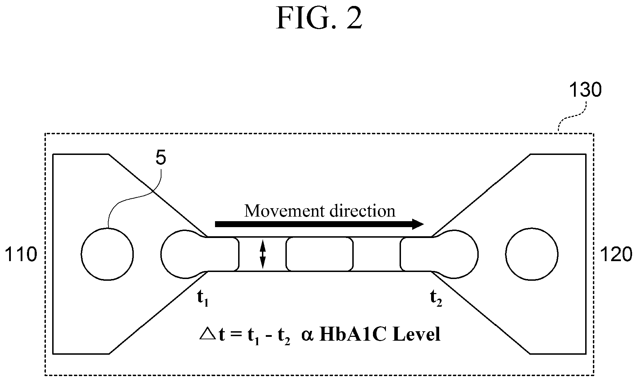

A principle of determining the degree of glycation through the microchannel passage time of red blood cells will be described with reference to FIG. 2.

FIG. 2 is a diagram illustrating a microchannel passage process of a red blood cell according to an embodiment of the present disclosure.

Referring to FIG. 2, a red blood cell 5 in blood has elasticity like a normal cell, and accordingly, is capable of passing through a passage with a width smaller than a general size of the red blood cell by reducing a diameter of the body. According to this principle, the red blood cell has a characteristic of changing shape and size according to the original stiffness in order to pass through a minute blood vessel together with blood. However, when hemoglobin in the red blood cell is combined with a sugar component in the blood and thus glycated, the physical stiffness of the red blood cell increases, and thus, elasticity of the red blood cell is reduced and the red blood cell becomes harder.

The red blood cell with increased stiffness due to the influence of glycated hemoglobin needs more time to pass through even a passage of the same width. The glycated hemoglobin measuring apparatus according to the present embodiment is to measure glycated hemoglobin using a proportional relationship between a passage time (t=t1-t2) and a glycated hemoglobin level (HbA1C level).

Furthermore, in the present embodiment, in order to more precisely measure the time taken for the small-sized red blood cell to pass through the microchannel, a parameter measured through a configuration of an electrical circuit is additionally used.

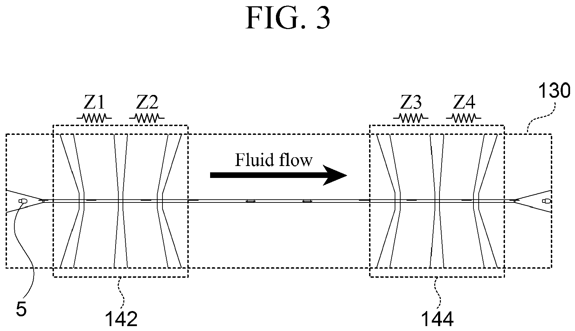

Hereinafter, a detailed configuration of the glycated hemoglobin measuring apparatus 100 according to the present embodiment will be described with reference to FIG. 3.

FIG. 3 is a diagram illustrating a more enlarged and detailed configuration of the microchannel 130 of the apparatus according to FIG. 1. Referring to FIG. 3, in the microchannel 130 of the glycated hemoglobin measuring apparatus 100 according to the present embodiment, electrodes patterned in a direction different from, preferably perpendicular to, a passage direction of a fluid are arranged at a predetermined interval.

Specifically, intervals between the electrodes may be formed at the same interval at positions in contact with the microchannel, and the width of the electrode may become wider away from the contact position, and thus, a configuration of a circuit may be further simplified.

In the present embodiment, the electrodes are directly in contact with the fluid passing through the microchannel and allow current to flow. At this time, the current flowing between the electrode and the electrode is affected by the ion concentration of an electrolyte on the microchannel, and specifically, when the red blood cell 5 is positioned between the electrode and the electrode, an impedance value changes. That is, the glycated hemoglobin measuring apparatus 100 according to the present embodiment may measure a microchannel passage time of the red blood cell 5 as a change in the impedance value of the set of electrodes 140.

Specifically, in order to calculate the channel passage time, a microchannel passage start time and a microchannel passage end time may be measured, and the microchannel passage time of the red blood cell may be calculated using a difference of the times.

Accordingly, in the present embodiment, the set of electrodes 140 may include a first set of electrodes 142 on the side of the inlet 110 and a second set of electrodes 144 on the side of the outlet 120.

The first set of electrodes 142 measures the passage start time of the red blood cell as the change the impedance value, and the second set of electrodes 144 measures the passage end time of the corresponding red blood cell as the change the impedance value, to calculate the microchannel passage time of the red blood cell as a difference between a time based on the change of the impedance value of the first set of electrodes 142 and a time based on the change of the impedance value of the second set of electrodes 144.

Furthermore, in the present embodiment, each set of electrodes may include a plurality of electrodes. An electrode for measuring the passage start time and another electrode for measuring the passage end time may be with a minimum configuration, but it is also possible to configure the circuit with more electrodes and measure additional information such as the size and type of a passing cell.

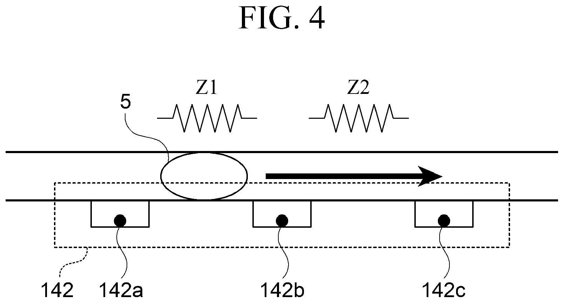

Specifically, referring to FIGS. 3 and 4, each of the first and second set of electrodes 142 and 144 may include three consecutive electrodes.

That is, each of the three consecutive electrodes in the set of electrodes 142 and 144 generates a change in two impedance values, and a change in each of impedance values Z1, Z2, Z3, and Z4 may occur in the side of the inlet 110 and the side of the outlet 120.

Referring to FIG. 4, the impedance values Z1 and Z2 between three electrodes 142a, 142b and 142c in the first set of electrodes 142 may be sequentially changed according to the passage order of the red blood cells 5.

Conversely, although not shown, the impedance values Z3 and Z4 of the three electrodes in the second set of electrodes 144 may also be sequentially changed according to the passage order of the red blood cells.

Depending on a degree to which the impedance values are sequentially changed, the size and type of the passing cell may be inferred, and through this, a more accurate glycated hemoglobin level may be calculated.

Furthermore, in the present embodiment, the glycated hemoglobin measuring apparatus 100 may more sensitively measure a minute change in an impedance value by using a change in the electrical equilibrium state between the respective two resistances of the first and second set of electrodes and the internal resistance of the glycated hemoglobin measuring main body 1000.

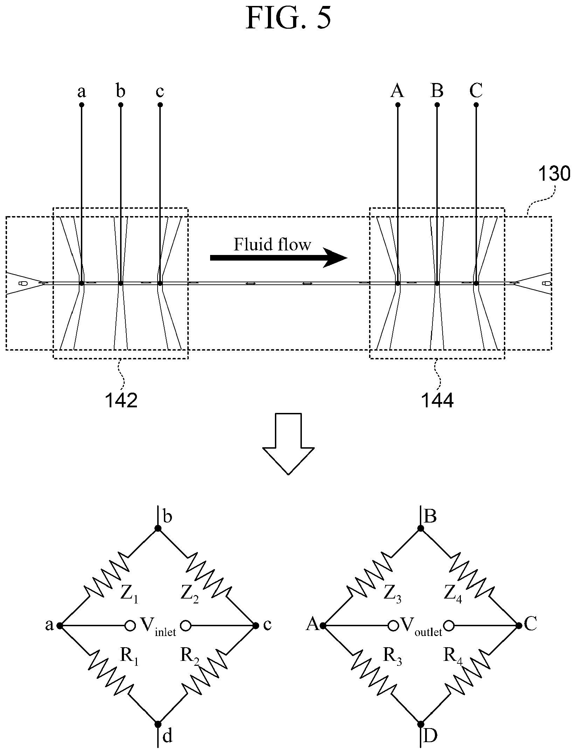

Referring to FIG. 5, the impedance generated through the relationship between the electrode and the channel according to FIG. 3 may be configured to have the electrical equilibrium state in a bridge structure according to the circuit configuration of a glycated hemoglobin measuring main body 1000.

Specifically, each of the impedances Z1, Z2, Z3, and Z4 on the microchannel of FIG. 3 may correspond to each impedance of a Wheatstone bridge circuit.

The bridge circuit may have an equilibrium state according to the characteristic of the set of electrodes, the physical distance, and the circuit configuration, and each impedance value may have a relationship as shown in the following Equation.

### [Equation 1]

### Zl*R2=Z2*Rl, Z3*R4=Z4*R3

In the circuit of FIG. 5, when each impedance value is in the equilibrium state as in Equation 1, no current may flow between nodes a and c and between nodes A and C, but as described above, when red blood cells are positioned between the electrode and the electrode, a potential difference may be generated by an instantaneous change in the impedance value.

That is, when a red blood cell is positioned at a node between the electrodes, a potential difference is generated according to the changed impedance, and in the present embodiment, an electrode passage time of the red blood cell may be determined through the time and size of the minute current flowing to the nodes a and c and the nodes A and C. Through this, the minute change in the impedance value may be measured.

Specifically, changes in a first impedance and a second impedance formed between two consecutive electrodes of the first set of electrodes 142, and changes in a third impedance and a fourth impedance formed between two consecutive electrodes of the second set of electrodes 144 may be more sensitively measured by using the change in the electrical equilibrium state in the Wheatstone bridge circuit, and the microchannel passage time may be calculated using the time based on the change.

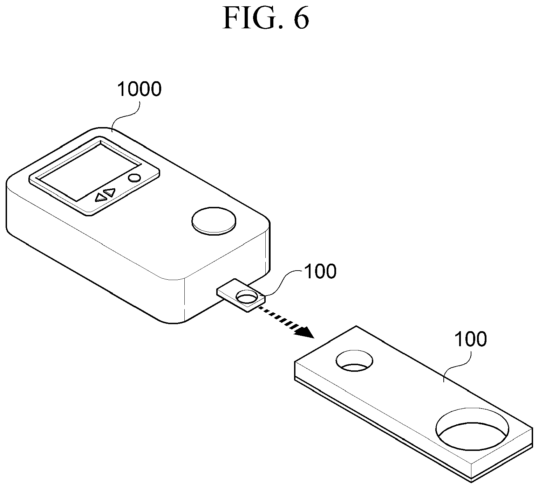

Referring to FIG. 6, in the present embodiment, the glycated hemoglobin measuring apparatus 100 may include the main body 1000 for outputting measured results, as a sensor chip configured in the above-described type of substrate by combining the chip, and it is also possible to miniaturize the chip and the entirety of the measuring equipment.

The main body 1000 provides power to allow current to flow through the microchannel 130 and the electrode configuration in the glycated hemoglobin measuring apparatus 100, measures the time based on the change of the impedance value, calculates the glycated hemoglobin level using the measured time difference, and outputs the glycated hemoglobin level through a panel.

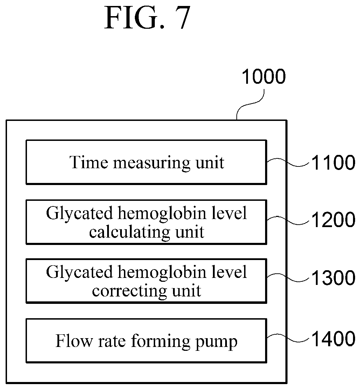

Specifically, referring to FIG. 7, the glycated hemoglobin apparatus main body 1000 may include a time measuring unit 1100, a glycated hemoglobin level calculating unit 1200, a glycated hemoglobin level correcting unit 1300, and a flow rate forming pump 1400.

The time measuring unit 1100 may measure the microchannel passage time by using a difference between the time based on the change of the impedance value as the red blood cell in the blood passes through the first node on the microchannel 130 formed in contact with the set of electrodes 140 between the blood inlet 110 and the outlet 120, and the time based on the change of the impedance value at the second node of the microchannel 130.

In the present embodiment, the first node is a node on the first set of electrodes 142 and may be preferably determined between an electrode and another electrode or may be defined as a predetermined zone on a microchannel. The second node may also be determined on the second set of electrodes 144 in correspondence to the first node.

The time measuring unit 1100 calculates the microchannel passage time of the red blood cell as the difference between the time based on the change of the impedance value of the first set of electrodes 142 and the time based on the change of the impedance value of the second set of electrodes 144.

Specifically, the time measuring unit 1100 may calculate the microchannel passage time by using the difference between the time based on the change of the impedance value as the red blood cell is positioned between at least one pair of electrodes in the first set of electrodes 142 and the time based on the change of the impedance value as the red blood cell is positioned between at least one pair of electrodes in the second set of electrodes 144.

The glycated hemoglobin level calculating unit 1200 calculates the degree of glycation of blood by using the correlation between the microchannel passage time and the glycation of the red blood cell in the blood.

Because each red blood cell has a different probability of meeting and combining with a sugar component in the blood depending on a period in which the red blood cell is formed, the glycated hemoglobin level of each individual red blood cell may be different.

Specifically, glycated hemoglobin level data of individual red blood cell at the measurement time node may represent a specific data distribution, and a representative value indicating the characteristic of the distribution may differ depending on the glycated hemoglobin level.

In this regard, a difference in the representative value may be derived by a formula based on clinical data sufficiently secured as the correlation between the microchannel passage time and the glycated hemoglobin level, and the glycated hemoglobin level calculating unit 1200 finally calculates the degree of glycation of the blood using the induced correlation formula by utilizing the measurement data measured by the time measuring unit 1100.

In the present embodiment, the glycated hemoglobin level calculating unit 1200 continuously measures a movement time of red blood cells according to the order in which the red blood cells enter, so that the passage time of each individual red blood cell is measured even if several red blood cells enter at once, and finally, thereby calculating a correlation equation with high accuracy within a short time and calculating the degree of glycation.

Furthermore, in order to more accurately determine the glycated hemoglobin level in consideration of individual characteristic, the glycated hemoglobin level correcting unit 1300 for correcting the glycated hemoglobin level using a reference glycated hemoglobin level measured by a user visiting a clinic may be included. In this regard, additional information such as the size or type of a cell measured by the electrode may be used.

In addition, a structure for inducing the movement of red blood cells in the blood in a direction from the inlet 110 to the outlet 120 is formed in the microchannel of FIG. 2 to serve as the flow rate forming pump 1400. A mixed solution including blood and electrolyte moves from the inlet 110 to the outlet 120 through the flow rate forming pump 1400 in the order described with reference to FIG. 2. At this time, the measurement unit 1100 measures an electrical signal due to the impedance that changes as the cells move, and the calculating unit 1200 calculates the glycated hemoglobin level by using the measured value.

Additionally, it is also possible to determine a management status of the glycated hemoglobin of the user by using the data distribution formed by the glycated hemoglobin level data of the individual red blood cell described above. Red blood cells are present in the blood according to their average lifespan, and thus, the lifespan of red blood cell and the glycated hemoglobin level may be related to each other.

In other words, when diabetes or glycated hemoglobin is not specifically managed, or when regular management is maintained without any change in administration or surgical procedure in the existing management state, the frequency of red blood cells according to the glycated hemoglobin level is similar to the frequency of red blood cells according to the lifespan.

At this time, when the user starts administration to manage the glycated hemoglobin level, the glycated hemoglobin level in newly made red blood cells decreases, and the distribution of red blood cells with relatively low stiffness increases. These changes may appear as features of the corresponding distribution.

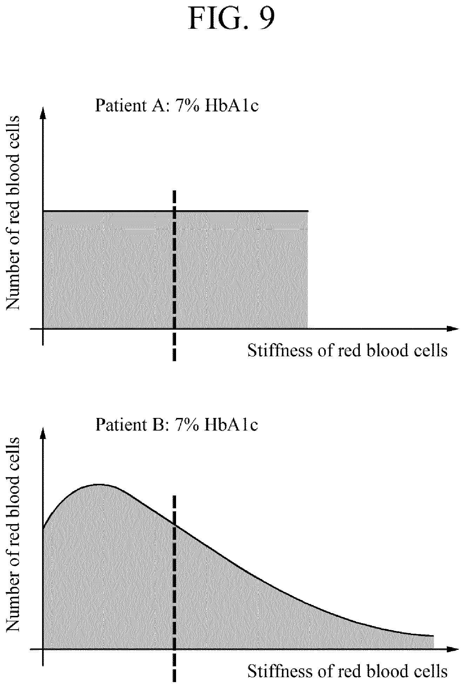

Referring to FIG. 9, even with the same average glycated hemoglobin level (7% HbA1c), a distribution graph of the number of red blood cells according to the stiffness of red blood cells may show a difference in the distribution as in the case of unmanaged patient A and managed patient B. That is, the similarity between the frequency of red blood cells according to the lifespan of the red blood cells decreases.

Therefore, the glycated hemoglobin measuring apparatus according to the present embodiment may objectively determine the health management status of the user through a change in the distribution of the calculated glycated hemoglobin level data of individual red blood cell, and more effectively prescribe and manage chronic diseases by presenting a health management strategy with the change in the distribution in addition to the current status as a representative value of the glycated hemoglobin level. In addition, an additional prescription in accordance with the characteristic of the user is possible by comparing the degree of change in the management status for each user.

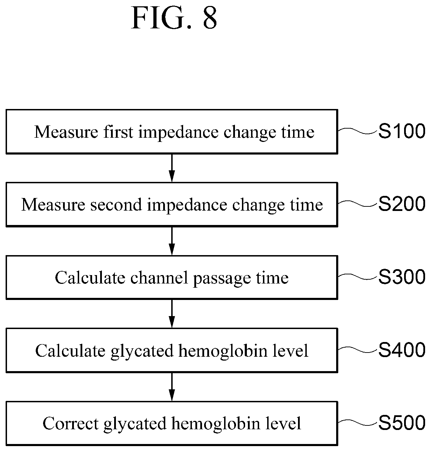

Hereinafter, a measurement method performed by the glycated hemoglobin measuring apparatus according to the present embodiment will be described with reference to FIG. 8.

Referring to FIG. 8, first, as a red blood cell in blood passes through at a first node on the microchannel 130 formed in contact with the set of electrodes 140 between the blood inlet 110 and the outlet 120, a time based on the change of an impedance value is measured (S100).

Then, a time based on the change of an impedance value is measured at a second node of the channel (S200).

In this regard, the first node may be determined as at least one position on a zone where the first set of electrodes 142 on the inlet side and the microchannel 130 contact each other, the second node may be determined as at least one position on a zone where the second set of electrodes 144 on the outlet side and the microchannel 130 contact each other, and first or second impedance time based on the change may be measured as a change in the impedance value as the red blood cell passing through the channel is positioned between a pair of electrodes in the first or second set of electrodes 142 or 144.

Next, a channel passage time is measured (S300) using a difference between the measured first impedance time based on the change of the first node and the measured second impedance time based on the change of the second node.

Next, the glycated hemoglobin level of the blood is calculated (S400) using the proportional relationship between the measured channel passage time and the glycation of red blood cells in the blood.

In addition, the calculated glycated hemoglobin level of the blood may be corrected based on a reference glycated hemoglobin level of the user to be used for clinical determination (S500).

As described above, according to the present disclosure, the degree of glycation may be easily measured using the change in the physical characteristics of red blood cells according to the glycation of the red blood cells.

In addition, the degree of glycation may be determined more stably in response to external and human factors compared to the measurement equipment of biochemical technique by calculating the hardness of each individual red blood cell through the time taken to pass through a minute channel.

In addition, the present disclosure may recognize a minute electrical change that occurs by the passage of the red blood cells through a simple circuit configuration, and through this, determine the degree of glycation of the red blood cells.

In addition, the present disclosure may be directly utilized for clinical diagnosis by correcting the glycated hemoglobin level measured by using an individual reference value.

Furthermore, various embodiments described herein may be implemented in a recording medium readable by a computer or a similar device using, for example, software, hardware, or a combination thereof.

According to a hardware implementation, the exemplary embodiments described herein may be implemented using at least one of application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, and electric units for performing other functions. In some cases, the exemplary embodiments described in the present specification may be implemented by a control module itself.

According to a software implementation, the exemplary embodiments such as procedures and functions described in the present specification may be implemented as separate software modules. Each of the software modules may perform one or more functions and operations described in the present specification. Software code may be implemented in software applications written in a suitable programming language. The software code may be stored in a memory module and may be executed by the control module.

The above description is merely illustrative of the technical idea of the present disclosure, and various modifications, changes, and substitutions may be made by those skilled in the art without departing from the essential features of the present disclosure.

Accordingly, the exemplary embodiments disclosed in the present disclosure and the accompanying drawings are not intended to limit the technical idea of the present disclosure but to describe the present disclosure, and the scope of the technical idea of the present disclosure is not limited by the exemplary embodiments and the accompanying drawings. The protection scope of the present disclosure should be interpreted by the following claims, and all technical ideas within the equivalent scope should be interpreted as being included in the scope of the present disclosure.

## Claims

1. A glycated hemoglobin measuring apparatus comprising:
an inlet into which a collected blood is introduced;
a microchannel having a predetermined width so that red blood cells in the blood introduced into the inlet pass in an individual unit;
an outlet through which the red blood cells that have passed through the microchannel are discharged; and
a plurality of set of electrodes formed in contact with the microchannel between the inlet and the outlet,
wherein the set of electrodes comprise a plurality of electrodes arranged at a predetermined interval according to a passage direction of the red blood cells passing through the microchannel.

2. The glycated hemoglobin measuring apparatus of claim 1, wherein
the set of electrodes include a first set of electrodes on an inlet side and a second set of electrodes on an outlet side, and
an impedance between at least one pair of electrodes in the first set of electrodes and an impedance between at least one pair of electrodes in the second set of electrodes change according to passage of the red blood cells.

3. The glycated hemoglobin measuring apparatus of claim 2, wherein
each of the first and second set of electrodes includes three continuous electrodes, and
an electrical equilibrium state of a first impedance and a second impedance formed between two consecutive electrodes of the first set of electrodes, and an electrical equilibrium state of a third impedance and a fourth impedance formed between two consecutive electrodes of the second set of electrodes change according to the passage of the red blood cells.

4. A glycated hemoglobin measuring apparatus comprising:
a time measuring unit measuring a microchannel passage time of red blood cells using a difference between a time based on the change of an impedance value as the red blood cells in blood pass through at a first node on a microchannel formed in contact with set of electrodes between a blood inlet and an outlet, and a time based on the change of an impedance value at a second node of the microchannel; and
a glycated hemoglobin level calculating unit determining a degree of glycation of the red blood cells in the blood based on the measured time.

5. The glycated hemoglobin measuring apparatus of claim 4, wherein
the set of electrodes include a first set of electrodes on an inlet side and a second set of electrodes on an outlet side, and
the time measuring unit calculates the microchannel passage time of the red blood cells by a difference between a time based on the change of an impedance value of the first set of electrodes and a time based on the change of an impedance value of the second set of electrodes.

6. The glycated hemoglobin measuring apparatus of claim 5, wherein
each of the first and second set of electrodes include a plurality of electrodes, and
the time measuring unit calculates the microchannel passage time of the red blood cells using a difference between a time based on the change of the impedance value when the red blood cells are positioned between at least one pair of electrodes in the first set of electrodes, and a time based on the change of the impedance value when the red blood cells are positioned between at least one pair of electrodes in the second set of electrodes.

7. The glycated hemoglobin measuring apparatus of claim 4, wherein
the glycated hemoglobin level calculating unit calculates the degree of glycation of the blood by using a proportional relationship between the microchannel passage time and glycation of the red blood cells in the blood.

8. The glycated hemoglobin measuring apparatus of claim 7, further comprising: a glycated hemoglobin level correcting unit correcting the degree of glycation of the blood by using a reference glycated hemoglobin level of a user.

9. The glycated hemoglobin measuring apparatus of claim 6, wherein
each of the first and second set of electrodes includes three continuous electrodes, and
the time measuring unit measures a change in the impedance value using a change in electrical equilibrium states of a first impedance and a second impedance formed between two continuous electrodes of the first set of electrodes, and electrical equilibrium states of a third impedance and a fourth impedance formed between two continuous electrodes of the second set of electrodes, and calculates the microchannel passage time using a time based on the change.

10. A glycated hemoglobin measuring method comprising:
measuring a time based on the change of an impedance value as red blood cells in blood pass through at a first node on a channel formed in contact with set of electrodes between a blood inlet and an outlet;
measuring a time based on the change of an impedance value at a second node of the microchannel;
measuring a microchannel passage time using a difference between a first impedance time based on the change of the first node and a second impedance time based on the change of the second node; and
calculating a glycated hemoglobin level according to the measured microchannel passage time.

11. The glycated hemoglobin measuring method of claim 10, wherein
the first node is determined as at least one position on a zone where a first set of electrodes on an inlet side and the microchannel contact each other, and
the second node is determined as at least one position on a zone where a second set of electrodes on an outlet side and the microchannel contact each other.

12. The glycated hemoglobin measuring method of claim 11, wherein the first or second impedance time based on the change is measured as a change in the impedance value as the red blood cells passing through the microchannel are positioned between a pair of electrodes in the first or second set of electrodes.

13. The glycated hemoglobin measuring method of claim 10, wherein the calculating of the glycated hemoglobin level includes calculating a degree of glycation of the blood using a proportional relationship between the microchannel passage time and glycation of red blood cells in the blood.

14. The glycated hemoglobin measuring method of claim 10, further comprising: correcting the calculated degree of glycation of the blood using a reference glycated hemoglobin level of a user.

15. The glycated hemoglobin measuring method of claim 10, wherein
each of the first and second set of electrodes includes three continuous electrodes,
the measuring of the microchannel passage time includes measuring a change in the impedance value using a change in electrical equilibrium states of a first impedance and a second impedance formed between two continuous electrodes of the first set of electrodes, and electrical equilibrium states of a third impedance and a fourth impedance formed between two continuous electrodes of the second set of electrodes, and
calculating the microchannel passage time by using a time based on the change of the impedance value.
